## Europäisches Patentamt
## European Patent Office
## Office européen des brevets

(11) Publication number: **0 013 534**
**A1**

(12) **EUROPEAN PATENT APPLICATION**

(21) Application number: **79810001.2**

(22) Date of filing: **11.01.79**

(51) Int. Cl.³: **A 61 M 15/00**

(43) Date of publication of application:
**23.07.80 Bulletin 80/15**

(84) Designated Contracting States:
**CH**

(71) Applicant: **SCHERICO LTD.**
**Töpferstrasse 5**
**CH-6004 Lucerne(CH)**

(72) Inventor: **Kistler, Frederic**
**Zürichstrasse 46**
**CH-6004 Lucerne(CH)**

(74) Representative: **Antony, Fritz, Dr. et al,**
**P.O. Box 601 Winkelriedstrasse 35**
**CH-6002 Lucerne(CH)**

(54) **Inhalation device.**

(57) Inhalation device (1) having a skirt (2) to receive an aerosol container (5) that discharges a metered dose of medicament through a longitudinal passage in a valve stem (6) each time the valve stem (6) is depressed, and a head-piece (3) having a mouth-piece (4) extending therefrom and a valve stem boss (8) adapted to receive said valve stem (6) in a discharge passageway (9) therein and having a discharge orifice (11).

In order to provide an audible signal to the patient on correct inhalation, indicating that he should actuate the container to receive the metered dose, the inhalation device (1), having an air-passageway through said skirt (2) and head-piece (3) to said mouth-piece (4), has a sounding device, in particular a thin metal membrane (12) with a vibratory reed or tongue (14) therein, inside the head-piece (3) or the adjacent part of the skirt (2), the reed or tongue (14) being adapted to sound only when air passes through the inhalation device during inhalation.

Fig. 1

# INHALATION DEVICE

This invention relates to improvements in inhalation devices for aerosol products contained in aerosol containers and in particular to an inhalation device especially suited for the oral administration, by inhalation,
of medicaments such as bronchodilators and corticosteroids.

On inhalation, these medicaments must reach the smallest
bronchial tubes in the lungs to get into contact with
the endothelial lining, where their medicinal effect
is needed  for example in the treatment of patients
suffering from obstructive bronchitis or asthma. However, many patients on long-term inhalation therapy
find it difficult to use inhalation devices for medicaments correctly and consequently do not obtain full
benefit from the medicament. Ideally, the patient should
exhale  as far as possible, hold the mouth-piece of
the inhalation device between his teeth, close his lips
and inhale as deeply as possible through the mouth, and,
while inhaling, actuate the aerosol container within the

0013534

inhalation device in order to obtain a metered dose of medicament. If the patient correctly carries out this procedure, the medicament is carried down into the bronchial tubes of the lungs where its therapeutic effect is needed. Unfortunately, many patients find it difficult to synchronise actuating the aerosol container and inhaling the medicament expelled therefrom; they may not inhale sufficiently or even not inhale at all. The result is that the active substance is not inhaled into the lungs but lodges in the mouth or throat instead, where it is ineffective for the treatment of asthma or obstructive bronchitis.

It has therefore been proposed ("The Lancet", December 4 1976) to modify the inhalation device by attaching a whistle or siren to the outside and by blocking any airspaces between the aerosol container and the adjacent part of the housing of the inhalation device. When the patient correctly uses this modified device, the whistle or siren sounds as the air passes through it, and the resulting audible signal indicates to the patient that he should press the container in order to receive the metered dose.

This modified device unfortunately suffers from a number of disadvantages: it is not simple to manufacture; it is too bulky; and the externally attached whistle or siren can get damaged or broken off if the inhalation device (with its aerosol container) is simply carried in the pocket or a case along with other articles.

It is therefore an object of the present invention to provide an improved inhalation device that generates an audible signal on correct inhalation, and in particular an improved inhalation device that, in a preferred embodiment, avoids or mitigates the aforesaid disadvantages by having its audible signal generating means in a position where it cannot be damaged in normal use, by being simple and cheap to manufacture, and by being no more bulky than the standard known inhalation device.

Accordingly, the invention provides an inhalation device comprising a body having a skirt and a head-piece, a mouth-piece extending from said head-piece and in communication therewith, an air-passageway extending through at least part of said body to said mouth-piece and providing a path for air to flow into the mouth-piece upon inhalation through the mouth-piece, and an audible signal generating means, said skirt being adapted to receive and locate an aerosol container of the type having a composition under pressure therein and having metering valve means including a valve stem and associated metering means, the valve stem having an axial discharge tube extending therethrough for discharge of a metered dose upon actuation of said metering valve means by depression of said valve stem, said head-piece having actuating means for said metering valve means and a discharge passageway leading to a discharge orifice, said actuating means being engageable with the valve stem upon location of the container within the skirt and operable to actuate the metering valve means whereby a metered dose is discharged through the valve stem discharge tube and the discharge passageway and discharge
orifice into the mouth-piece, wherein said audible signal
generating means is located in said air-passageway within
the body and is actuatable upon inhalation through said
mouth-piece when said inhalation device is in engagement
with said container.

The actuating means for said metering valve means is preferably a valve stem boss adapted to receive the valve stem
within a discharge passageway therein, said discharge
passageway having means for limiting entry therein of said
valve stem and a discharge orifice communicating with said
discharge passageway and adapted to discharge said metered dose through said mouth-piece.

Although the valve stem boss is designed to grip the
valve stem of the container, the container is relatively easily dislodged (e.g. if the inhalation device
is carried loose in the pocket or a case with other ar-
ticles) unless the skirt is long enough to accommodate and
protect the container. The skirt therefore should not
simply overlap the top part of the container but extend at
least half way, preferably at least three quarters of the
way, along the container, and thus accommodate the container and hold it snugly inside.

The audible signal generating means (for convenience
hereinafter referred to as "sounding device") is located
preferably inside the head-piece or the adjacent part of
the skirt where it will neither interfere with the movement
of the container necessary to actuate the valve and discharge the metered dose nor obstruct the discharged metered dose, thus preferably in the air path upstream from the discharge orifice. A convenient position is between the space reserved for the container and the valve stem boss; in particular the sounding device can be placed adjacent to and most preferably abutting against the valve stem boss, near the junction of the head-piece and skirt.

In order to provide an air path through the body to the mouth-piece, a small hole can be left opposite the mouth-piece near the junction of the skirt and head-piece, and the sounding device can be located between this hole and the mouth-piece. However, preferably one or more channels are provided between the container and the skirt as a path for air to pass into the head-piece and then out of the mouth-piece on inhalation.

The sounding device is preferably a thin membrane having a vibratory reed or tongue therein. Such a sounding device is cheap and simple to produce, can easily be inserted into and retained in the inhalation device, and does not hinder the normal use of the inhalation device. Moreover, because it fits inside the inhalation device, it does not add to the bulk of the inhalation device and is not subject to damage in normal use.

- 6 -

A particularly preferred embodiment of the invention
will now be described with reference to the accompanying drawings, in which:

Fig. 1 is a side elevational view of a preferred form
of inhalation device, partly in section, and

Fig. 2 is an enlarged sectional view of the inhalation
device taken along line 2-2 of Fig. 1.

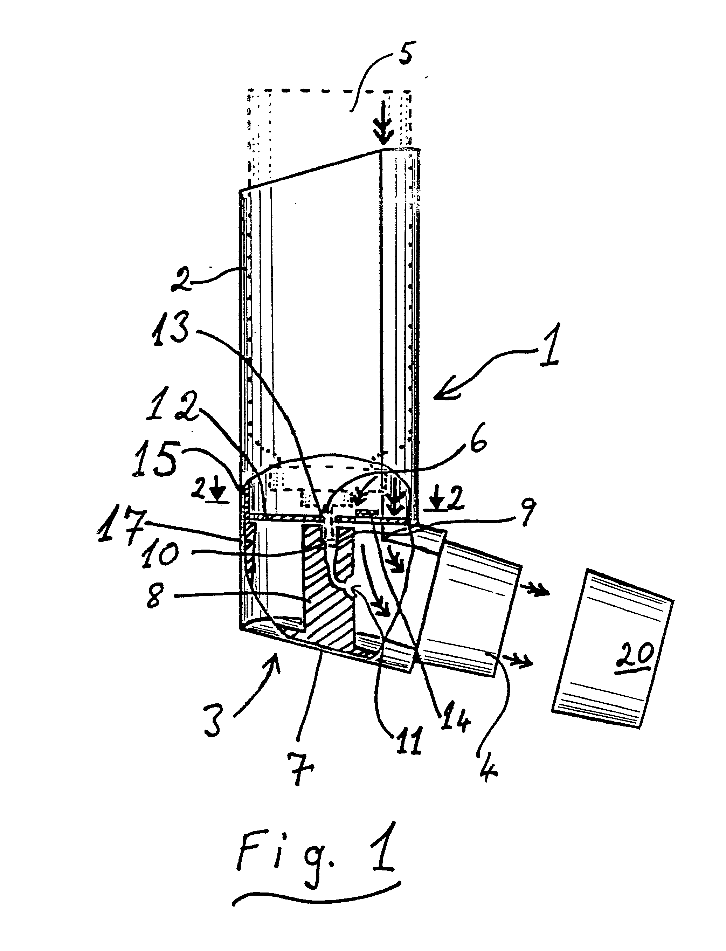

The inhalation device 1, which is preferably made of
rigid or semi-rigid plastic material, comprises a
skirt  2 and a head-piece 3, together constituting a
body,          and a mouth-piece 4. The skirt 2 is designed to receive an aerosol container 5 of standard
type, which upon depression of a valve stem 6 dispenses
metered doses of medicament through an axial discharge
tube therein. In Fig. 1, the container 5 is indicated by
broken lines, since its presence is not necessary to the
general description of the invention but only to how
the invention is used. The head-piece 3 of the inhalation
device 1 has a top portion 7 from which a valve stem
boss 8 depends. Valve stem boss 8 is adapted to receive
and grip valve stem 6 of container 5 by means of a longitudinal discharge passageway 9 therein. This discharge
passageway 9 has one or more abutments 10 therein designed to limit the penetration of the valve stem 6 into the boss 8 when pressure is exerted on the opposite end of the container 5. The discharge   passageway 9 communicates with a discharge orifice 11 substantially on the longitudinal axis of the mouth-piece 4. The mouth-piece 4 can be closed by a cap 20 when the inhalation device 1 is not being used.

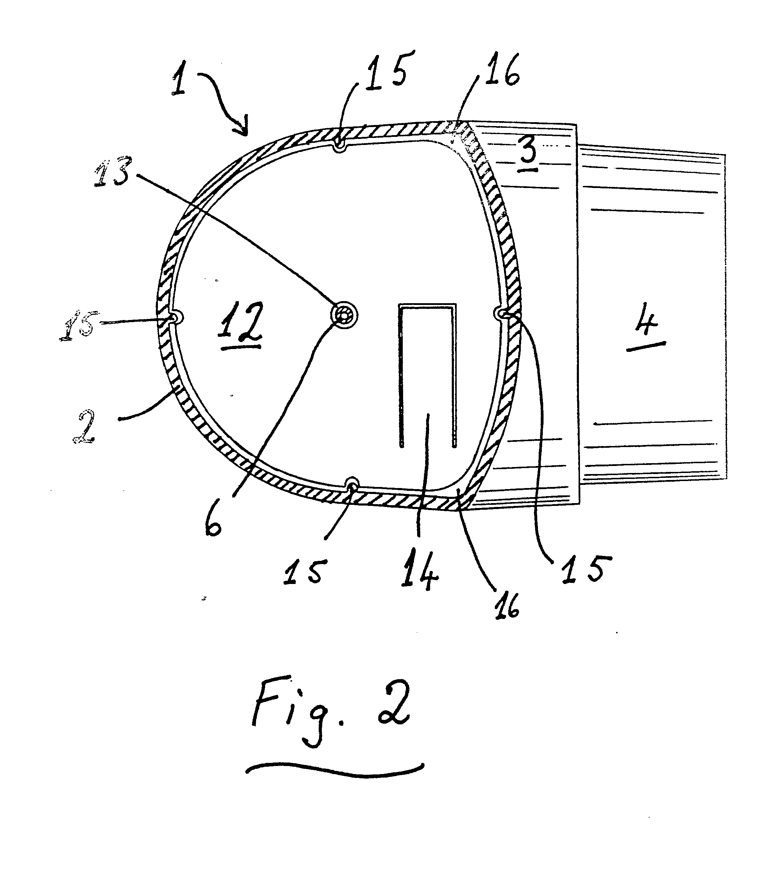

The inhalation device 1 contains a thin metal membrane 12 abutting against the boss 8. This membrane 12, which is shown in elevation in Fig. 2, has an aperture 13 at its center to receive the valve stem 6, and in particular has a U-shaped or rectangular reed or tongue 14 stamped in it. The reed or tongue 14 is preferably set at a small angle to the plane of the membrane 12 and bent towards the container 5, so that it will vibrate only when the air flows first past the membrane 12 and then out through the mouth-piece 4, as in inhalation. This provides a "one-way" sounding device.

The skirt 2 of the inhalation device 1 preferably has small ribs 15 protruding therefrom inside, these having the dual function of strengthening the skirt 2 and limiting the sideways movement of the container 5. The membrane 12 is correspondingly notched to accommodate these ribs 15. Normally the membrane 12 will reside comfortably inside the inhalation device 1 without further support, but if desired a number of abutments 17 can be provided in the head-piece 3 of the inhalation device 1, e.g. as continuations of the ribs 15. The membrane 12 fits snugly inside the skirt 2 but has sufficient air gaps around it or through it to allow good inhalation by the patient; small gaps, as at 16, are sufficient. If necessary, a small aperture or a number of small apertures may be made in the membrane 12. However, the air gaps in or around the membrane must be sufficiently constricted to force an air flow past the reed or tongue 14 during inhalation and thus activate it. The container 5 is circular in cross-section, so an air path (generally shown by double-headed arrows in Fig. 1) is provided between the container 5 and the skirt 2 in the direction of the air gaps 16, and this extends past the membrane 12 and through the head-piece 3 and mouth-piece 4.

The membrane 12 should be sufficiently rigid to allow insertion into the inhalation device 1 without suffering deformation. It should be made of a readily available cheap metal that can be readily stamped to the desired form, e.g. a moderately hard metal such as hard or semi-hard brass. Brass sheet 0.1 to 0.2 mm. thick is very suitable. Reed or tongue 14 may be for example 5 to 20 mm. long, preferably 7 to 15 mm. long and in particular about 9 to 12 mm. long; it may be 2 to 7 mm. wide, more preferably 3 to 5 mm. wide, in

particular about 3.5 to 4 mm. wide; and it may be about 2 to 4 times as long as it is wide, preferably about 2.5 to 3 times. In order to produce the desired "one-way" sounding effect as mentioned above, the reed or tongue 14 is preferably set towards container 5 at a small angle to the plane of the membrane 12. The angle may be for example about 2 to 4 degrees, preferably about 3 degrees; for a reed or tongue 9 to 12 mm. long, its free end should be about 0.5 mm. out of the plane of the membrane. If necessary, more than one reed or tongue 14, if desired tuned to different pitches, can be incorporated into the membrane 12.

The membrane 12 may be if necessary pigmented or metal-plated on one surface so that the two surfaces have a different appearance; this provides a visual check that it has been inserted correctly.

The membrane 12 can be stamped out on a suitable machine, which can simultaneously set the reed or tongue 14 at the necessary angle  for example by means of a slight protrusion on the punch. It can then be inserted into the inhalation device 1.

In using the inhalation device 1, the patient inserts the container 5 into the skirt 2;  he empties his lungs, puts

the mouth-piece 4 into his mouth, grips it with his teeth and closes his lips. He then deeply inhales through the mouth, and presses the container 5 (to actuate it) when he hears the sound from the membrane 12. He continues inhaling for as long as possible after actuating the container, and then holds his breath as long as possible. The present inhalation device can also be used by a doctor in demonstrating to a patient the importance of synchronizing inhalation through the mouth and actuating the container, so that the patient can better use an unmodified device.

CLAIMS

1.      An inhalation device comprising a body having a skirt and a head-piece, a mouth-piece extending from said head-piece and in communication therewith, an air-passageway extending through at least part of said body to said mouth-piece and providing a path for air to flow into the mouth-piece upon inhalation through the mouth-piece, and an audible signal generating means, said skirt being adapted to receive and locate an aerosol container of the type having a composition under pressure therein and having metering valve means including a valve stem and associated metering means, the valve stem having an axial discharge tube extending therethrough for discharge of a metered dose upon actuation of said metering valve means by depression of said valve stem, said head-piece having actuating means for said metering valve means and a discharge passageway leading to a discharge orifice, said actuating means being engageable with the valve stem upon location of the container within the skirt and operable to actuate the metering valve means whereby a metered dose is delivered through the valve stem discharge tube and the discharge passageway and discharge orifice into the mouth-piece, wherein said audible signal generating means is located in said air-passageway within the body and is actuatable upon inhalation through said mouth-piece when said inhalation device is in engagement with said container.

2.    A device as claimed in claim 1 wherein said actuating means for said metering valve means comprises a valve stem boss adapted to receive the valve stem within a discharge passageway therein, said discharge passageway having means for limiting entry therein of said valve stem and a discharge orifice communicating with said discharge passageway and adapted to discharge said metered dose through said mouth-piece.

3.    A device as claimed in claim 2 wherein said means generating an audible signal is located in said air-passageway upstream of said discharge orifice, preferably upstream of said boss between the space reserved for the container and said boss.

4.    A device as claimed in claim 3 wherein said means generating an audible signal abuts against the valve stem boss.

5.    A device as claimed in any of claims 1 to 4 wherein said means generating an audible signal is responsive to an air-flow in one direction only past said means and out  through said mouth-piece.

6.      A device as claimed in any of claims 1 to 5 wherein the sound-generating part of said means generating an audible signal is a vibratory reed or tongue.

7.      A device as claimed in claim 6 wherein said means generating an audible signal is a thin membrane having the vibratory reed or tongue therein, preferably a metal membrane, especially a brass membrane 0.1 to 0.2 mm. thick.

8.      A device as claimed in claim 7 wherein said reed or tongue is from 7 to 15 mm. long, 3 to 5 mm. wide, and preferably from 2.5 to 3 times as long as it is wide.

9.      An inhalation device comprising a body having a skirt and a head-piece, a mouth-piece extending from said head-piece and in communication therewith, an air-passageway extending through at least part of said body to said mouth-piece and providing a path for air to flow into the mouth-piece upon inhalation through the mouth-piece, and an audible signal generating means, said skirt being adapted to receive, locate and accommodate an aerosol container of the type having a composition under pressure therein and having metering valve means including a valve stem and associated metering means, the valve stem having an axial discharge tube extending therethrough for discharge of a metered dose upon actuation of said metering valve means by depression of said valve stem, said head-piece having a valve stem boss adapted to receive said valve stem within a discharge passageway therein, and said valve stem boss having means for limiting entry of said valve stem into said valve stem boss and a discharge orifice communicating with said discharge passageway and adapted to discharge said metered dose through said mouth-piece, said air path being defined in said skirt by channels provided between said skirt and the space reserved for the container, wherein said audible signal generating means is a thin metal membrane located between the space reserved for the container and said boss and abutting against said boss, and having a vibratory reed or tongue therein, said reed or tongue being bent toward the container so as to vibrate only when air flows inwardly through the skirt and outwardly through the mouth-piece.

10. A device as claimed in any of claims 1 to 9 in engagement with an aerosol container therefor.

Fig. 1

**Fig. 2**

| | DOCUMENTS CONSIDERED TO BE RELEVANT | | CLASSIFICATION OF THE APPLICATION (Int. Cl.3) |
|---|---|---|---|
| Category | Citation of document with indication, where appropriate, of relevant passages | Relevant to claim | |
| X | <u>DE - A - 2 803 993</u> (CALZADA Y COMPANIA)<br>* claims 1, 4; pages 2 to 6;<br>  fig. 5 *<br><br>-- | 1,3-6,<br>9,10 | A 61 M  15/00 |
| | <u>GB - A - 994 755</u> (RIKER LABORATORIES)<br>* claims 1 to 6; fig. 2, 3, 5 *<br><br>-- | 1,2,<br>9,10 | |
| | <u>FR - A - 1 345 733</u> (LABORATOIRES<br>   J.BERTHIER)<br>* fig. 2 *<br><br>-- | 1,2,<br>9,10 | TECHNICAL FIELDS SEARCHED (Int.Cl.3)<br><br>A 61 M  15/00 |
| A | <u>DE - A  - 2 341 346</u> (FISONS LTD.)<br>* page 3, lines 5 to 13; claims 1, 13 *<br>&  GB - A - 1 392 945<br>&  FR - A - 2 196 818<br><br>---- | | |

| X | The present search report has been drawn up for all claims | | |
|---|---|---|---|
| Place of search | | Date of completion of the search | Examiner |
| Berlin | | 22-08-1979 | DROPMANN |

EPO Form 1503.1  06.78